# FASCICULE DE BREVET EUROPEEN

(11) **EP 1 301 587 B1**
(45) Date de publication et mention de la délivrance du brevet: **14.11.2007**
(21) Numéro de dépôt: 01949567.0
(22) Date de dépôt: 28.06.2001
(51) Int. Cl.: C12N 1/00, C12N 1/18, C12N 1/38, C12C 12/04, C12G 3/08

(54) **PROCEDE DE CULTURE DE MICRO-ORGANISMES EN CONDITIONS REDUCTRICES OBTENUES PAR UN FLUX GAZEUX**
VERFAHREN ZUM KULTIVIEREN VON MIKROORGANISMEN UNTER REDUZIERENDEN BEDINGUNGEN MITTELS EINER GASSTRÖMUNG
METHOD FOR CULTURING MICRO-ORGANISMS IN REDUCING CONDITIONS OBTAINED BY A GAS STREAM

(30) Priorité: 04.07.2000 FR 0008684
(43) Date de publication de la demande: 16.04.2003
(73) Titulaire: L'AIR LIQUIDE, Société Anonyme pour l'Etude et l'Exploitation des Procédés Georges Claude, 75007 Paris (FR)
(72) Inventeur: CACHON, Rémy, F-21000 Dijon (FR); CAPELLE, Nathalie c/o ENSBANA, F-21000 Dijon (FR); DIVIES, Charles, F-21000 Dijon (FR); PROST, Lucie, F-75006 Paris (FR)
(74) Mandataire: Mellul-Bendelac, Sylvie Lisette
(86) Numéro de dépôt international: PCT/FR2001/002067
(87) Numéro de publication internationale: WO 2002/002748

(56) Documents cités:
- WO-A-96/41888
- GIOVANELLI GABRIELLA ET AL: "Kinetics of grape juice fermentation under aerobic and anaerobic conditions." AMERICAN JOURNAL OF ENOLOGY AND VITICULTURE, vol. 47, no. 4, 1996, pages 429-434, XP000992676 ISSN: 0002-9254
- VONKTAVEESUK PHENJUN ET AL: "Stimulation of the rate of L-lactate fermentation using Lactococcus lactis IO-1 by periodic electrodialysis." JOURNAL OF FERMENTATION AND BIOENGINEERING, vol. 77, no. 5, 1994, pages 508-512, XP000990917 ISSN: 0922-338X

## Description

La présente invention est relative à la culture de micro-organismes en conditions réductrices obtenues au moyen d'un flux gazeux.

Elle concerne plus particulièrement la production modifiée et/ou contrôlée de produits alimentaires par fermentation utilisant des levures en conditions réductrices telles que boissons alcoolisées, produits laitiers, etc., ferments ou levains, levures-aliments, ferments probiotiques, extraits de levures.

Elle est plus précisément relative à un procédé permettant de diminuer le potentiel d'oxydo-réduction lors de la culture de micro-organismes, notamment lors de fermentations utilisant des levures pour la préparation des produits précités.

Les oxydo-reductions sont des étapes essentielles dans les réactions de l'anabolisme et du catabolisme cellulaire, pour lesquelles le sens des échanges est déterminé par le potentiel d'oxydo-réduction (Eh). Celui-ci est un paramètre d'état des fermentations et sa variation modifie l'environnement physico-chimique des micro-organismes dont il influence les activités métaboliques et la physiologie.

Les levures sont utilisées très largement pour la fabrication des boissons fermentées.

Chez la levure, le bilan redox de la fermentation est normalement équilibré par la production d'éthanol. De plus, la levure utilise une partie des sucres fermentescibles pour synthétiser de la biomasse et des produits secondaires dont le principal est le glycérol, troisième constituant du vin après l'éthanol et l'eau. La production de ce dernier dépendra de la quantité de co-enzymes réduits disponibles pour les biosynthèses. Lors de la fermentation des sucres, la formation de l'éthanol et du glycérol est ainsi essentielle pour maintenir la balance redox.

D'autres produits sont également synthétisés en quantités suffisamment importantes pour modifier les caractéristiques sensorielles du produit final. Il s'agit essentiellement des acides organiques. La formation des acides organiques tels que le succinate ou l'acétate lors de la fermentation a également une influence sur la balance rédox. Le succinate est formé par voie oxydative via le citrate et l'α-cétoglutarate ou par voie réductrice via l'oxaloacétate, si bien que les conditions de fermentation restent essentielles. L'acétate est également formé à partir de l'acétaldéhyde par une aldéhyde déshydrogénase.

La levure se caractérise aussi par la production de composés volatils intervenant dans les arômes fermentaires, à savoir, les alcools supérieurs, les aldéhydes et les esters. Ces produits sont issus des métabolismes glucidiques, lipidiques et azotés, et de ce fait, leur production est également dépendante d'une modification possible de la répartition des flux lors d'une variation du potentiel redox.

A partir du métabolisme des sucres, la souche de levure synthétise aussi des oligosaccharides de réserve comme le glycogène et le tréhalose, souvent en réponse à un stress environnemental. Ces deux oligosaccharides peuvent s'accumuler quand le carbone est épuisé dans le milieu mais aussi lorsque la souche ne dispose plus de source azotée ou soufrée ou encore lors d'un stress thermique ou osmotique.

Les sucres de réserve jouent un rôle important pour améliorer la stabilité et le séchage des levures, de ce fait, leurs concentrations constituent un paramètre critique dans la production de « poudres de levures actives » (Active Dry Yeast en anglais). En effet, pour faire face aux températures élevées du cycle de séchage, des fractions de glycogène sont utilisées pour fournir l'énergie de maintenance à la cellule alors que le tréhalose est lui, utilisé comme un facteur stabilisant les membranes. Pendant le séchage, les levures peuvent accumuler de grandes quantités de tréhalose (10-15% du poids sec) et d'ailleurs, la qualité des levures séchées seraient en relation directe avec le contenu en tréhalose cellulaire. La survie à la déshydratation est corrélée avec la synthèse de tréhalose et lors de la réhydratation, le tréhalose est dégradé. En fait, le tréhalose se fixe sur les groupements phosphates des membranes et en remplaçant l'eau il stabilise ces dernières lors de la déshydratation. En plus d'agir comme des facteurs de stockage et protecteurs, ces sucres peuvent jouer également un rôle dans la progression du cycle cellulaire à faible taux de croissance sous une limitation en carbone.

Compte tenu des connaissances actuelles, des variations du potentiel redox semblent pouvoir modifier la répartition des produits et éventuellement des intermédiaires métaboliques.

Jusqu'à présent, pour modifier le potentiel d'oxydo-réduction d'un milieu de fermentation, on procède soit par traitement thermique (comme la pasteurisation) soit par ajout de molécules d'addition oxydo-réductrices (telles l'acide ascorbique, des sulfites......).

Néanmoins, ces méthodes peuvent modifier les caractéristiques des produits obtenus et ne peuvent pas être mises en oeuvre systématiquement dans les domaines agroalimentaire (oenologie par exemple), pharmaceutique ou vétérinaire en raison des normes imposées.

On peut également signaler les travaux de Vonktaveesuk et al parus dans Journal of Fermentation and Bioengineering en 1994, vol 77, pages 508-512, qui concernent les bactéries lactiques, et qui semblent suggérer que la baisse du potentiel redox par l'hydrogène possède un effet négatif sur l'objectif que s'était fixé les auteurs, notamment un ralentissement de la fermentation.

L'invention a ainsi pour objectif de remédier aux inconvénients précités et de fournir un procédé de culture de micro-organismes permettant de modifier les flux métaboliques dans les cellules en faisant varier le potentiel redox du milieu.

L'invention a aussi pour objectif de fournir un procédé de fermentation utilisant des levures dans la perspective d'une application agroalimentaire en particulier en oenologie, ou d'une application pharmaceutique ou vétérinaire, faisant intervenir des moyens non toxiques et non préjudiciables pour les produits finaux.

Un autre objectif de l'invention est de fournir un tel procédé permettant d'accélérer ou de simplifier les procédés de fabrication par fermentation.

L'objectif de l'invention est aussi de fournir un procédé permettant d'améliorer les durées de conservation en particulier des ferments ou levains.

Un objectif de la présente invention est également de fournir des produits fermentés tels que des boissons présentant un faible degré d'alcool (vins, bières, etc.) et/ou des propriétés organoleptiques modifiées.

Un autre objectif de la présente invention est encore de fournir des ferments, levures-aliments ou extraits de levures présentant des propriétés améliorées notamment du point de vue conservation, nutritionnel et/ou organoleptique.

A ces fins, l'invention a pour objet un procédé de culture de micro-organismes conforme à la revendication ci-après.

Le procédé selon l'invention va être décrit plus en détails ci-après.

Les inventeurs ont mis en évidence de manière tout à fait surprenante, que l'on pouvait faire varier le potentiel redox d'un milieu de culture de micro-organismes au moyen d'un flux gazeux réducteur comparé à l'air tel qu'un flux gazeux (différent de l'air) contenant de l'hydrogène, et que les conditions réductrices ainsi obtenues permettaient de modifier les flux métaboliques lors de la culture.

Ils ont ainsi montré que l'on pouvait orienter les flux métaboliques vers la production de composés spécifiques et/ou modifier, de manière contrôlée, les propriétés des produits obtenus.

De manière plus spécifique, les inventeurs ont montré que l'on pouvait modifier les flux métaboliques ou la cinétique de prise de mousse lors de la fermentation de levures, dans le cadre d'applications oenologiques.

Ils ont encore montré que l'on pouvait augmenter la quantité de sucres de réserve produite lors de la culture de micro-organismes pour la production de biomasse.

Ils ont également mis en évidence que l'on pouvait augmenter la viabilité des micro-organismes en conditions réductrices telles que définies et prolonger la durée de conservation des ferments obtenus.

Selon l'invention, par « conditions réductrices », on se réfère aux conditions obtenues à l'aide d'un flux gazeux qui est réducteur comparé à l'air, comprenant de l'hydrogène, mis au contact d'un milieu de culture, permettant d'abaisser le potentiel d'oxydoréduction dudit milieu de culture par rapport à la valeur qu'il aurait en l'absence dudit flux gazeux, c'est-à-dire à l'air, toutes choses étant égales par ailleurs.

L'invention couvre ainsi les cultures en milieux réducteurs proprement dits (on a abaissé le potentiel redox en dessous de 0) mais également le cas où le flux gazeux permet d'abaisser le potentiel d'oxydoréduction d'un milieu initialement oxydant, même si le potentiel final atteint à l'aide dudit flux gazeux reste positif en soi (on est alors au final toujours en milieu oxydant).

On rappelle que les valeurs du potentiel redox dépendent notamment de la composition du milieu de culture et de son pH, la référence utilisée pour apprécier la baisse du potentiel redox obtenue conformément à l'invention, présentant la même composition de milieu à pH semblable.

Selon le procédé de l'invention, les conditions réductrices telles que définies ci-dessus sont obtenues au moyen d'un gaz réducteur comprenant de l'hydrogène.

Il est composé d'hydrogène seul ou en mélange, avec le cas échéant de plus un ou plusieurs autres gaz appelés ici «gaz complémentaire(s) acceptable(s) du point de vue de la culture ».

Le gaz complémentaire peut ainsi être choisi parmi les gaz inertes, notamment l'argon, l'hélium, mais aussi parmi l'oxygène, le dioxyde de carbone et le protoxyde d'azote et les mélanges en toutes proportions d'un ou plusieurs de ces gaz ; le gaz complémentaire peut être constitué d'un seul gaz ou d'un mélange de gaz.

Il est considéré comme « acceptable du point de vue de la culture » en ce sens qu'il n'interfère pas de manière négative avec celle-ci et permet donc un développement satisfaisant voire amélioré, des micro-organismes.

Il est en outre choisi parmi les gaz répondant aux normes et autorisations du domaine d'application considéré (par exemple, oenologie, denrées alimentaires, produits pharmaceutiques ou vétérinaires, etc.). Ceci s'entend bien sur des normes du moment connues sachant qu'elles évoluent en permanence en autorisant régulièrement l'arrivée et l'utilisation de nouveaux composés (voir par exemple les dérogations actuellement accordées en France pour l'utilisation d'ozone).

Le gaz complémentaire est de préférence sélectionné parmi le dioxyde de carbone et l'oxygène ainsi que leurs mélanges.

Lorsqu'il s'agit d'un mélange, le flux gazeux contient de préférence au moins 0,5% en volume d'hydrogène, plus préférentiellement entre 3 et 50% en volume d'hydrogène.

Pour des raisons de facilité de mise en oeuvre et de sécurité, la teneur en hydrogène est préférentiellement choisie inférieure à 5%.

Comme on l'aura compris à la lecture de ce qui précède, la composition du flux gazeux peut varier selon les souches mises en oeuvre et les applications visées ainsi que selon les contraintes de coût éventuellement imposées.

Conformément à l'invention, le procédé est réalisé selon les processus de culture classiquement utilisés pour les micro-organismes considérés.

Il peut s'agir en particulier de fermentation, en utilisant notamment les levures, ou encore d'autres processus de croissance de micro-organismes selon les applications choisies.

Des moyens pour mettre le milieu de culture en contact avec le flux gazeux précité sont en outre prévus.

Le gaz selon l'invention peut être appliqué, avant et/ou pendant la réalisation de la croissance des micro-organismes, par tous moyens connus.

Le procédé selon l'invention peut être mis en oeuvre de manière continue ou discontinue, ce dernier mode étant souvent préféré d'un point de vue industriel.

Comme démontré plus en détails plus loin dans la présente demande, le procédé selon l'invention permet de modifier les flux métaboliques des micro-organismes. Par « modification des flux métaboliques », on entend l'orientation contrôlée de la production lors d'une culture de micro-organismes, c'est-à-dire l'obtention de produits spécifiques éventuellement aux dépens d'autres produits normalement obtenus, mais également la modification des caractéristiques de ces flux, notamment du point de vue de la vitesse de production, de la montée en pression le cas échéant, etc.....

En d'autres termes, le procédé permet d'orienter et de contrôler lesdits flux métaboliques lors de la culture des micro-organismes de manière à obtenir au final une composition différente de celle qu'on obtiendrait en réalisant la même culture dans les conditions classiques, éventuellement accompagnée de la production de substances spécifiques nouvelles qui ne sont pas habituellement obtenues lors de la production classique, c'est-à-dire sans contact avec le gaz réducteur tel que défini selon l'invention.

Le procédé de l'invention permet également de modifier les flux métaboliques au niveau des caractéristiques de la réaction. De manière typique, on peut citer le cas de fermentations utilisant des levures en récipient fermé (par exemple bouteille) dont la cinétique de prise de mousse peut être améliorée en conditions réductrices comme décrites précédemment.

La modification des flux métaboliques peut également correspondre à une accumulation des sucres de réserve, en particulier tréhalose et glycogène, dans les cellules produites.

La présente invention pourra par ailleurs adopter une ou plusieurs des caractéristiques décrites en liaison avec les revendications 2 à 9 ci-après

On décrit ci-après des applications plus spécifiques de l'invention qui ne doivent toutefois pas être considérées comme limitatives et ne sont données qu'à titre illustratif.

Selon un mode de réalisation de l'invention, le procédé est appliqué à des micro-organismes du type utilisé en oenologie, pour la préparation de boissons alcoolisées non gazeuses (sans prise de mousse) telles que boissons à base de vins ou boissons distillées.

Il s'agit généralement de fermentations utilisant des levures du genre *Saccharomyces,* principalement réalisées en cuve (récipient ouvert) typiquement de manière discontinue.

Lorsque l'on se place en conditions réductrices telles que définies selon l'invention, on produit par exemple des boissons à teneur en glycérol plus élevée par comparaison aux mêmes types de boissons produites dans les conditions classiques, c'est-à-dire sans abaissement du potentiel redox du milieu.

L'augmentation de la production de glycérol se fait aux dépens de la production d'éthanol. On peut ainsi obtenir directement des boissons faiblement alcoolisées non gazeuses en évitant la désalcoolisation par un processus d'extraction, employé jusqu'à présent pour produire de telles boissons.

Le procédé selon l'invention s'applique donc notamment à la production de boissons à degré alcoolique réduit, par exemple boissons à base de vins faiblement alcoolisées. Dans ce cas, on préfère maintenir une teneur en éthanol d'au moins 5% en volume pour restituer les arômes et saveurs du vin, appréciés par les consommateurs, du fait de la rétention des composés volatils à l'origine de ces propriétés organoleptiques, dans l'éthanol.

L'invention peut cependant être appliquée à la production de boissons à degré alcoolique inférieur au seuil précité auquel cas, on peut produire des boissons faiblement alcoolisées avec de nouvelles propriétés organoleptiques.

Le procédé selon l'invention s'applique également à la production de boissons qui, à degré alcoolique équivalent, ont des propriétés organoleptiques améliorées, par exemple dans le domaine des boissons distillées.

Le procédé de l'invention présente l'avantage de permettre une récupération sensiblement totale et la non dégradation des composés non volatils ainsi que l'absence de formation de mauvais goûts liés au procédé de traitement, notamment pour la désalcoolisation classique.

Le procédé selon l'invention permet ainsi de contrôler les caractéristiques des boissons produites par fermentation, en particulier du point de vue des propriétés organoleptiques et de leur degré d'alcool. Il permet notamment de conserver ou renforcer les propriétés organoleptiques habituelles d'une boisson tout en en diminuant son degré alcoolique. Dans d'autres cas, il permet de produire des boissons présentant de nouvelles caractéristiques organoleptiques ou de textures non accessibles par les procédés classiques. On peut citer par exemple l'obtention de vins à teneur en glycérol élevée qualifiés de vins « gras ».

Le procédé selon l'invention est ainsi applicable à tout type de fermentation utilisant des levures ou autres micro-organismes de la même espèce que les levures où la diminution de la production d'éthanol et/ou l'augmentation de la production de glycérol sont recherchées.

Selon une variante de mise en oeuvre, le procédé est appliqué à des fermentations de levures réalisées en récipient fermé (par exemple bouteille ou tonneau) pour la production de boissons avec prise de mousse.

Il s'agit par exemple de fermentation de levures principalement du genre *Saccharomyces,* utilisée pour la production de vins mousseux, bières ou cidres.

Dans ce cas, en conditions réductrices telles que définies selon l'invention, on peut améliorer la cinétique de la prise de mousse en accélérant la montée en pression dans la bouteille.

On peut ainsi préparer des boissons de type champagnes, vins mousseux, pétillants de raisin, bières ou cidres, par un procédé plus facile et plus rapide.

Selon d'autres modes de réalisation de l'invention, le procédé est appliqué à des micro-organismes pour la préparation de ferments, levures-aliments ou encore extraits de levures, utilisés dans le domaine alimentaire mais également dans le domaine médical ou vétérinaire.

A titre d'exemples, on peut citer la culture d'espèces des genres *Saccharomyces* et *Candida* pour la production de ferments ou levains de panification ; la culture d'espèces des genres *Saccharomyces, Kluyveromyces* et *Candida* pour la production de levures-aliments notamment à usage pharmaceutique ou vétérinaire, et d'extraits de levures ; la culture de bactéries des genres *Lactococcus, Leuconostoc, Lactobacillus* et *Streptococcus thermophilus* pour la production de bactéries lactiques utilisées dans l'industrie laitière.

Lorsqu'on se place en conditions réductrices telles que définies conformément à l'invention, on produit par exemple davantage de sucres de réserve, notamment tréhalose et glycogène.

La teneur en sucres de réserves est un facteur important et même un paramètre critique dans la production de « poudres de levures actives » (Active Dry Yeast) qui sont obtenues par un procédé de déshydratation desdites levures.

On observe parallèlement une bonne viabilité des microorganismes dans les conditions réductrices de l'invention.

Le procédé décrit permet donc d'améliorer la production de telles levures déshydratées.

Il est ainsi applicable dans le domaine de la diététique où les levures sèches alimentaires sont utiles en tant qu'ingrédients alimentaires naturels, riches en protéines, vitamines du groupe B et minéraux.

Il est également applicable en pharmacie et parapharmacie où les levures sèches enrichies peuvent être incorporées dans des compléments nutritionnels, en particulier par la forte biodisponibilité des oligo-éléments qu'elles contiennent, et où les extraits de levures sont utiles en fermentation pharmaceutique mais aussi en microbiologie notamment pour la préparation de milieux de croissance.

Le procédé de la présente invention est encore applicable au domaine de l'alimentaire où les levures autolysées sont des ingrédients particulièrement bien adaptés à l'aromatisation notamment pour la préparation de snacks aromatisés ou de biscuits salés et où les extraits de levures sont des ingrédients traditionnels des bouillons, potages, etc. mais aussi des ingrédients riches en facteurs de croissance, en peptides et en acides aminés utiles pour les procédés de culture de microorganismes.

Le procédé selon l'invention est applicable à toute industrie utilisant des levains vivants telle que l'oenologie, la brasserie, la cidrerie, la panification mais également la pharmacie, la parapharmacie, le vétérinaire notamment avec l'essor des agents probiotiques.

L'invention est ainsi relative à tous les produits obtenus par le procédé décrit précédemment.

L'invention est illustrée à l'aide des exemples donnés ci-après, à titre non limitatif, en référence aux dessins dans lesquels :
- la figure 1 est un diagramme montrant la production d'éthanol et de glycérol obtenue pour différents potentiels redox (Eh) dans le cas d'une fermentation continue sur milieu minimum, en référence à l'exemple 1 ;
- les figures 2a à 2d et 3a à 3d sont des courbes montrant respectivement les rendements en éthanol et en glycérol observés en l'absence de gaz et sous trois bullages différents (engendrant quatre potentiels redox différents) au cours d'une fermentation alcoolique, en référence à l'exemple 2 ;
- les figures 4, 5 et 6 représentent les courbes montrant respectivement l'évolution de la production d'éthanol, de la pression dans la bouteille et du degré alcoolique (paramètres déterminants d'une prise de mousse) en fonction du temps, dans le cas d'une fermentation en bouteille avec prise de mousse, en référence à l'exemple 3 ;
- les figures 7, 8 et 9 représentent des courbes rapportant respectivement le potentiel redox (Eh), le suivi de la quantité de sucre résiduel et le suivi de la production de glycérol au cours du temps (paramètres annexes d'une prise de mousse) dans le cas d'une fermentation en bouteille avec prise de mousse, en référence à l'exemple 3 ;
- les figures 10 et 11 sont des diagrammes illustrant l'analyse quantitative des sucres de réserve, glycogène et tréhalose, en fonction du potentiel redox (Eh) respectivement sur milieu minimum et sur milieu jus de raisin, en référence à l'exemple 4 ;
- les figures 12 et 13 sont des courbes montrant la viabilité des cellules au cours de leur conservation respectivement dans de l'eau physiologique et dans le vin, en référence à l'exemple 5.

### EXEMPLES

### I - Effet des conditions réductrices selon l'invention sur la valeur du potentiel d'oxydoréduction d'un milieu de fermentation.

Pour cette étude, on a utilisé trois types d'atmosphères, à savoir azote seul, mélange azote/hydrogène à raison de 4% d'hydrogène et hydrogène seul.

On a observé l'effet d'un bullage de ces différents gaz sur la valeur du potentiel redox (Eh) d'un milieu minéral ayant la composition suivante convenable pour la croissance de *Saccharomyces cerevisiae :*

### Composition du milieu minéral :

| Quantités pour 1 litre d'eau distillée | |
|---|---|
| (NH₄)₂SO₄ | 5g |
| KH₂PO₄ | 3g |
| MgSO₄,7H₂O | 0,5g |
| EDTA | 15mg |
| ZnSO₄, 7H₂O | 4,5mg |
| CoCl₂, 6H₂O | 0,3mg |
| CaCl₂, 2H₂O | 4,5mg |
| FeSO₄, 7H₂O | 3mg |
| NaMoO₄, 2H₂O | 0,4mg |
| H₃BO₃ | 1mg |
| KCl | 0,1mg |
| Silicone antimoussant | 0,025ml |
| Ergostérol | 10mg |
| Tween 80 | 420mg |
| Ethanol 1mM | 46mg |
| Glucose | 23g |
| Biotine | 0,05mg |
| Calcium pantothénate | 1mg |
| Acide nicotinique | 1mg |
| Inositol | 25mg |
| Thiamine HCl | 1mg |
| Acide para-aminobenzoïque | 0,2mg |
| Pyridoxine HCl | 1mg |
| pH = 4,1 | |

On a également observé l'effet de ces gaz sur milieu jus de raisin correspondant à une application oenologique.

Dans un premier temps, on a observé la valeur du Eh sur le milieu stérile (c'est à dire non ensemencé) et dans un deuxième temps sur milieu ensemencé.

On fait buller dans le milieu de fermentation le gaz ou le mélange de gaz jusqu'à fixer la valeur du Eh. Ces expérimentations sont réalisées à 25°C et au pH du milieu considéré qui reste constant.

Le tableau 1 ci-dessous rapporte les valeurs du potentiel Redox (Eh) obtenues sur milieu minimum.

**Tableau I**

| | Azote | Azote/hydrogène | Hydrogène |
|---|---|---|---|
| Milieu stérile | +250 mV | -200 mV | -300 mV |
| | (pH = 4.13) | (pH = 4.14) | (pH = 5) |
| Milieu ensemencé (*) | +100 à +70 mV | -100 mV | -300 mV |
| | (pH = 5) | (pH = 5) | (pH =5) |

| | | | |
|---|---|---|---|
| (*) souche *Saccharomyces cerevisiae* CBS 8066 | | | |

Le tableau 2 indique lui les valeurs du potentiel Redox (Eh) obtenues sur milieu jus de raisin.

**Tableau 2**

| | Azote | Azote/Hydrogène | Hydrogène |
|---|---|---|---|
| Jus de raisin stérile | +300 mV | -100 mV | -200 mV |
| | (pH = 3.01) | (pH = 3.04) | (pH = 3.02) |
| Jus de raisin ensemencé (**) | +100 à +300 mV | -100 mV | -200 mV |
| | (pH = 3) | (pH = 3) | (pH =3) |

| | | | |
|---|---|---|---|
| (**) souche *Saccharomyces cerevisiae* RC 212 | | | |

A titre comparatif, le potentiel du milieu jus de raisin stérile est de 400 mV en conditions classiques (absence de flux gazeux selon l'invention).

### II - Effet des conditions réductrices selon l'invention sur les flux métaboliques de la fermentation.

### EXEMPLE 1 : ETUDE SUR MILIEU MINIMUM.

On a utilisé la souche *Saccharomyces cerevisiae* CBS 8066 qui se développe sur un milieu minéral en glucose limitant et supplémenté en vitamines et acides gras insaturés correspondant à la composition donnée au paragraphe I. Les levures sont cultivées en anaérobiose, en continu, à une température constante de 30°C et un pH maintenu à 5. Les taux de dilution D (rapport du débit d'alimentation au volume du liquide) sont balayés de 0,05 h⁻¹ à 0,3h⁻¹.

Trois valeurs du Eh sont appliquées grâce à l'utilisation des différents gaz soit 100 mV, - 100 mV et -300 mV.

On étudie la modification des flux métaboliques, lors de la fermentation, par analyse quantitative de la distribution des flux métaboliques :

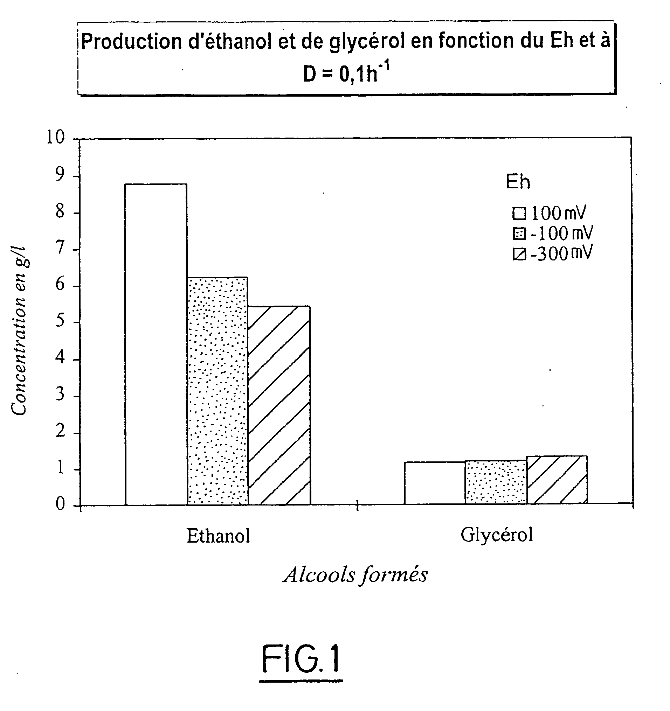

Les résultats observés sont rapportés en figure 1 qui présente la quantité (en g/l) d'éthanol et de glycérol pour les trois conditions de Eh précitées à D = 0,1h⁻¹. En milieu réduit, le flux carboné est dévié vers la production de glycérol au dépend de l'éthanol.

Les résultats obtenus lors du suivi de la stoechiométrie métabolite-substrat sont donnés au tableau 3.

Aux vus des résultats présentés dans ce tableau, on constate qu'en conditions réductrices (gaz hydrogène et mélange hydrogène/azote), la synthèse de glycérol est augmentée par rapport aux conditions oxydantes (sous azote) et ce, au dépend de celle de l'éthanol qui est fortement diminuée. Dans ces conditions, le ratio glycérol/éthanol est doublé lorsque l'on passe d'une fermentation sous azote à une fermentation sous hydrogène.

### EXEMPLE 2 : ETUDE SUR MILIEU JUS DE RAISIN.

On a utilisé une souche oenologique de *Saccharomyces cerevisiae* RC 212. La fermentation est menée en discontinu (batch) pendant 145 heures sur milieu jus de raisin contenant environ 165 g/l de sucres fermentescibles et la température est maintenue à 25°C. Il s'agit d'un milieu industriel dont les propriétés physico-chimiques et les concentrations en éléments nutritifs sont différentes du milieu minéral. Ce milieu présente la capacité de reproduire le phénomène à concentration élevée en sucres et avec un substrat se composant d'un mélange glucose-fructose équilibré. Cette expérience permet également de contrôler le phénomène en culture discontinue.

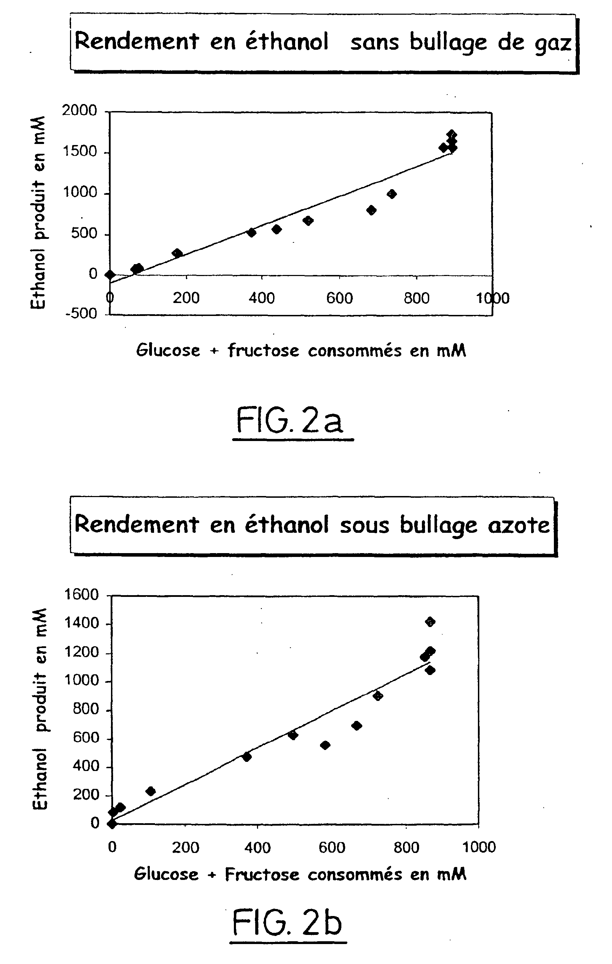

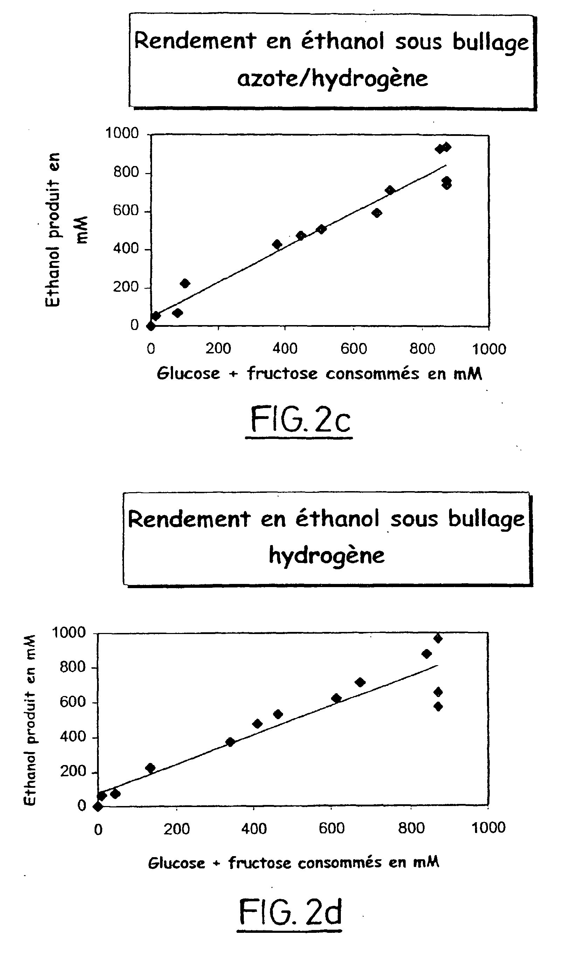

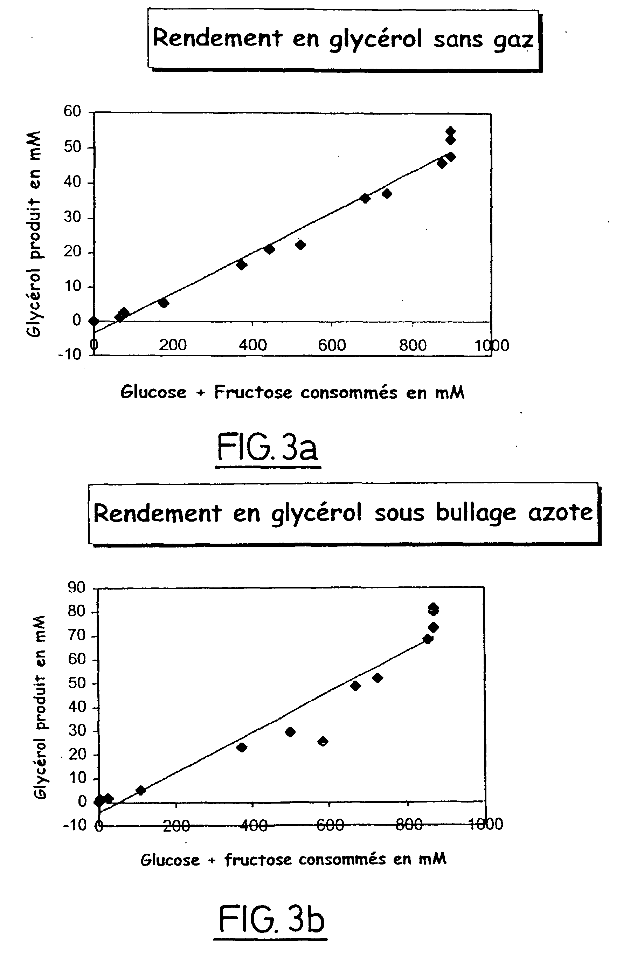

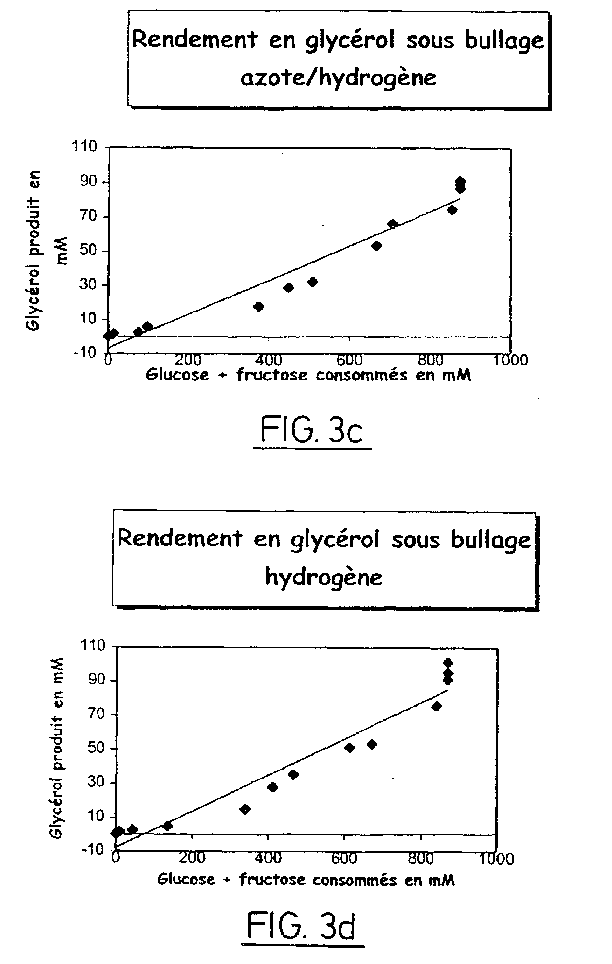

Quatre conditions de potentiel redox (Eh) sont testées en utilisant :
- un réacteur sans bullage de gaz (Figures 2a et 3a)
- un réacteur avec un bullage azote (Figures 2b et 3b)
- un réacteur avec un bullage azote/hydrogène (Figures 2c et 3c)
- un réacteur avec un bullage hydrogène (Figures d2 et 3d).

Au cours du temps, on a suivi la biomasse, le pH, l'évolution du Eh, la production d'éthanol et de glycérol ainsi que la quantité de sucres résiduels.

Les résultats présentés correspondent à une moyenne de 3 répétitions.

Ils sont illustrés sur les figures 2a à 2d pour l'éthanol et sur les figures 3a à 3d pour le glycérol.

Les concentrations en sucres dans les moûts étant variables (150 à 350 g/l de jus selon les cépages), il convient d'interpréter les résultats obtenus en prenant en compte les ratios éthanol formé/sucres fermentescibles (en mol/mol) et glycérol formé/sucres fermentescibles (en mol/mol) mais aussi le rapport glycérol/éthanol.

Cette analyse de la stoechiomètrie métabolites-sucres confirme, comme lors de l'étude sur milieu minimum en chémostat, le fait que l'on favorise la voie du glycérol au dépend de celle de l'éthanol lorsque la levure se retrouve en conditions réductrices.

Ces résultats montrent que dans le cadre d'une application en vinification, il est possible de produire une boisson fermentée à faible degré alcoolique, tout en maintenant (ou en augmentant) la production de glycérol qui intervient dans le corps et la rondeur des vins.

Le tableau 3 ci-dessous donne les résultats obtenus lors du suivi de la stoechiométrie métabolite - substrat dans le cas:
(1) d'une fermentation en chémostat à D = 0,1h⁻¹ sur milieu minimum (23 g/l de glucose) : exemple 1,
(2) d'une fermentation discontinue sur jus de raisin à 100 heures (165 g/l de sucres fermentescibles) : exemple 2.

Les rendements rapportés correspondent aux coefficients directeurs des droites de régression des figures 2a - 2d et 3a - 3d :
Figure 2a: y = 1,79x - 105,00 ; Rendement = 94 %
Figure 2b : y = 1,29x + 23,47 ; Rendement = 93 %
Figure 2c : y = 0,91 x + 45,92 ; Rendement = 96 %
Figure 2d : y = 0,85x + 72,42 ; Rendement = 88 %
Figure 3a : y = 0,06x - 3,51 ; Rendement =98 %
Figure 3b : y = 0,10x - 6,92 ; Rendement = 95 %
Figure 3c : y = 0,09x - 4,46 ; Rendement = 93 %
Figure 3d : y = 0,11 x - 8,08 ; Rendement = 94 %

A titre comparatif, ce tableau indique les valeurs rapportées par Michnick et al, 1997 (Yeast, vol. 13 , 783-793) et Oura, 1977 (Process Biochemistry, vol. 4 , 19-35) dans l'art antérieur, la seconde référence correspondant à une synthèse de 5 auteurs différents.

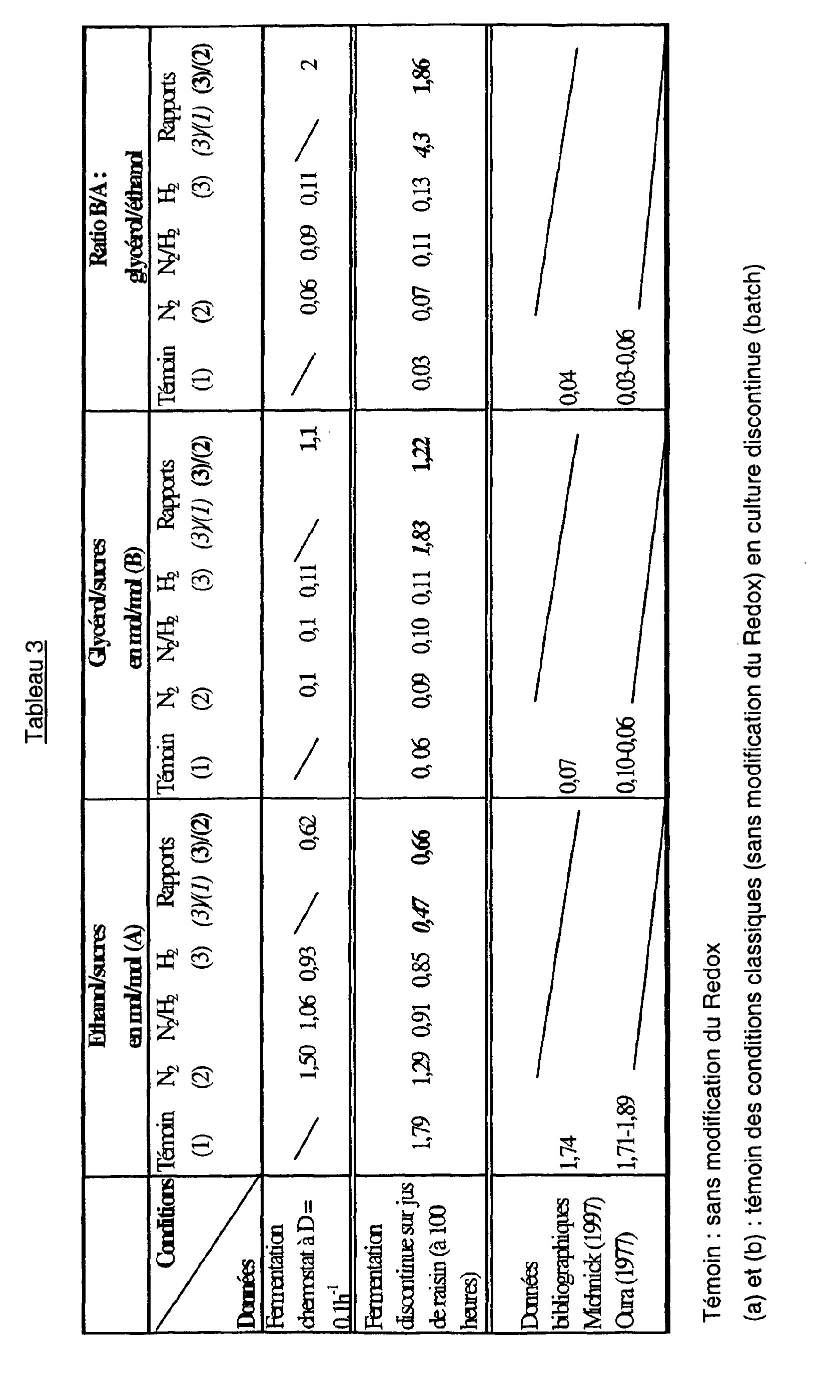

### EXEMPLE 3 : ETUDE SUR MILIEU CHAMPAGNE.

On utilise une souche oenologique de levure de tirage du genre *Saccharomyces.* Le milieu se constitue d'un vin de base additionné d'une liqueur enrichie en sucres.

Quatre conditions de Eh sont testées en utilisant :
- une mise en bouteille sans bullage de gaz,
- une mise en bouteille avec un bullage azote,
- une mise en bouteille avec un bullage azote/hydrogène,
- une mise en bouteille avec un bullage hydrogène.

Au cours du temps (6 semaines), on a suivi l'évolution du Eh, la production d'éthanol et de glycérol, la viabilité des cellules de levures, l'évolution de la pression ainsi que la quantité de sucres résiduels (glucose + fructose).

### - Suivi des paramètres déterminant pour évaluer la prise de mousse :

Il s'agit en fait de la mesure de la production d'éthanol, de l'évolution de la pression et du calcul du degré alcoolique atteint.

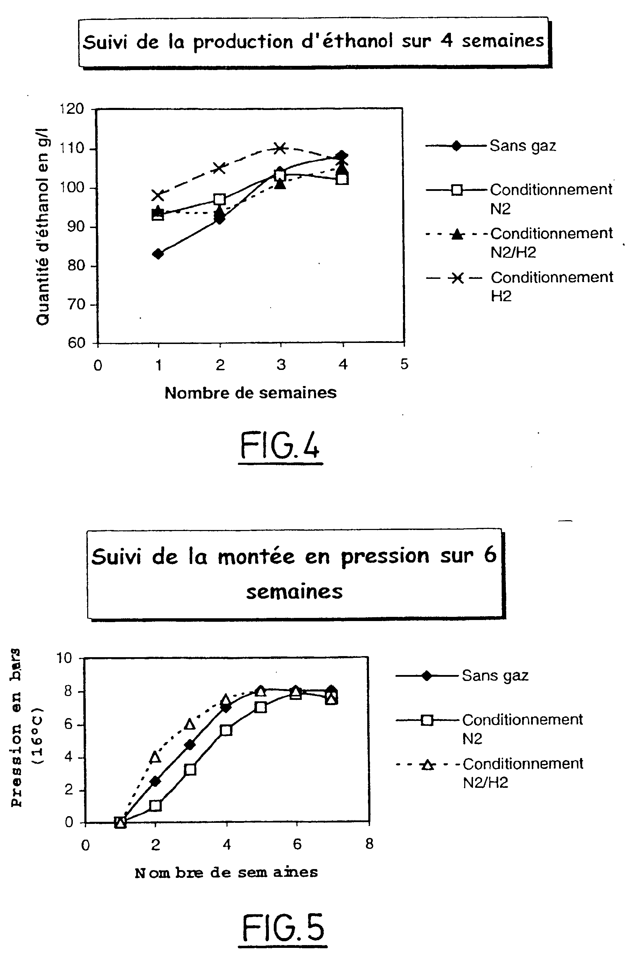

Aux vus des courbes présentées sur les figures 4, 5 et 6, on constate qu'au bout de 3 semaines, on atteint les 12,5° dans les bouteilles quelles que soient les conditions de Eh testées. On rappelle que selon la législation, cette valeur est exigible pour toute fabrication de Champagne.

### - Suivi des paramètres annexes :

Il s'agit là de la mesure du Eh et des quantités de glycérol et de sucres résiduels.

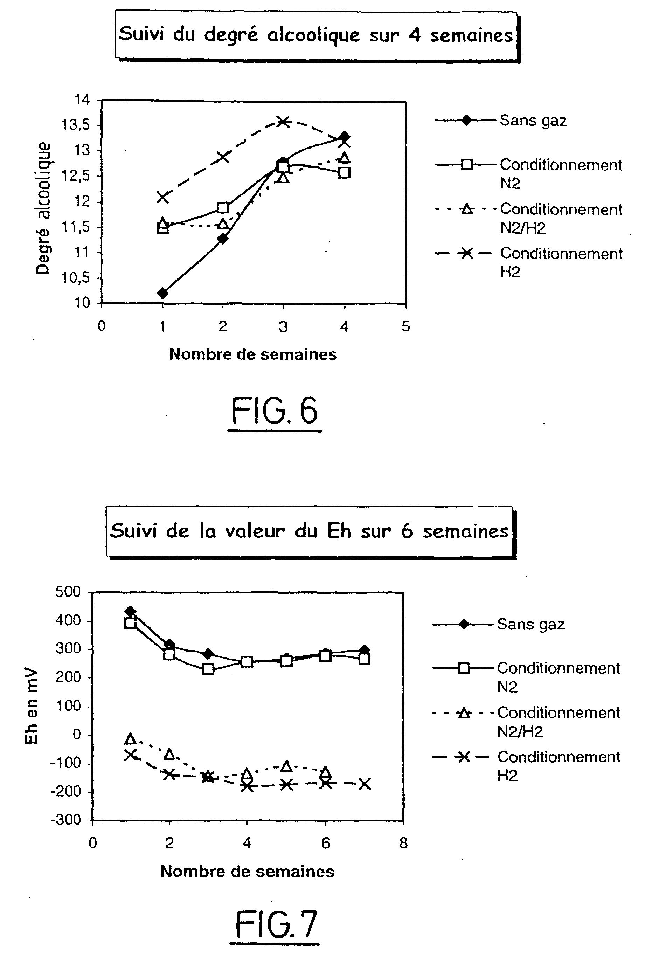

En référence à la figure 7, la mesure du Eh sur 6 semaines permet de conclure que la valeur se maintient dans les bouteilles au cours du temps.

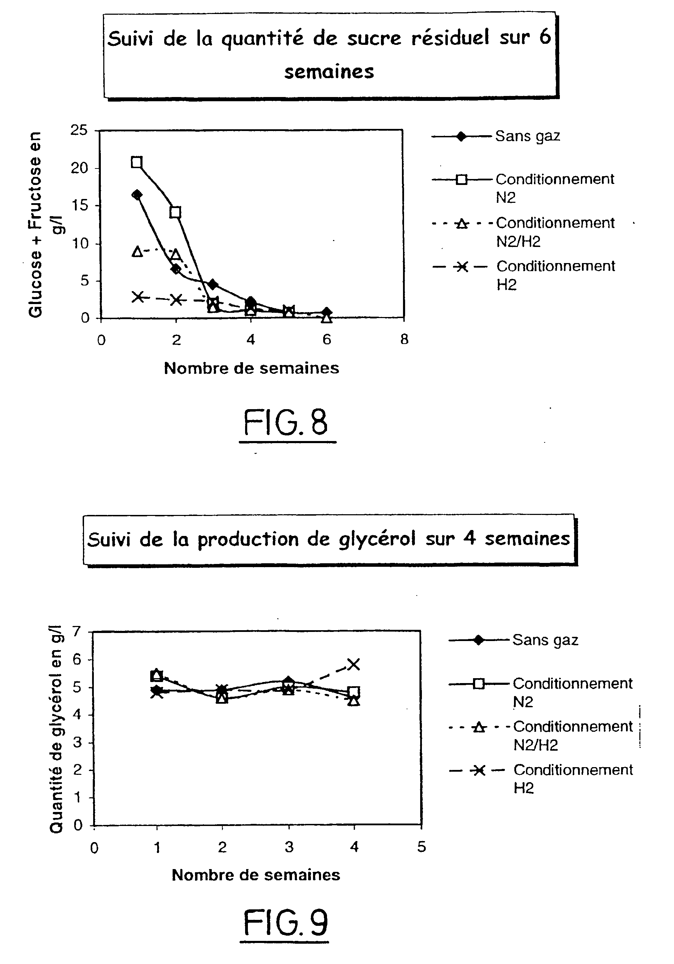

Pour ce qui est de la quantité des sucres résiduels, aux vus des courbes de la figure 8, selon les conditions de Eh testées la consommation du sucre, par la levure, ne se fait pas de la même manière. La souche placée en conditions réductrices consomme les sucres fermentescibles beaucoup plus rapidement que sous un environnement oxydant.

Enfin, en référence à la figure 9, on voit que la production de glycérol n'est pas sensiblement modifiée par les différentes conditions appliquées.

Cette étude montre le changement de la physico-chimie du produit, pendant la fermentation en bouteille, du fait de la modification du Eh en utilisant différentes conditions réductrices.

### III - Effet des conditions réductrices selon l'invention sur la teneur en sucres de réserve.

### EXEMPLE 4 : ETUDE SUR MILIEU MINIMUM

On a réalisé une croissance en batch de *Saccharomyces cerevisisae* CBS 8066 sur un milieu minéral limitant en glucose dont la composition correspond à celle donnée au 1, à 30°C et sous agitation à 300 rpm (rotation par minute).

Deux conditions de Eh sont testées:
- un réacteur sous un bullage azote,
- un réacteur sous un bullage hydrogène.

La croissance est menée pendant 14 heures, à la suite desquelles, un échantillon de cellules est prélevé pour effectuer le dosage du tréhalose et du glycogène selon le protocole établi par Parrou et François, 1997 (Analytical Biochemistry, vol. 248, 186-188).

Les résultats présentés correspondent à une moyenne de 2 dosages.

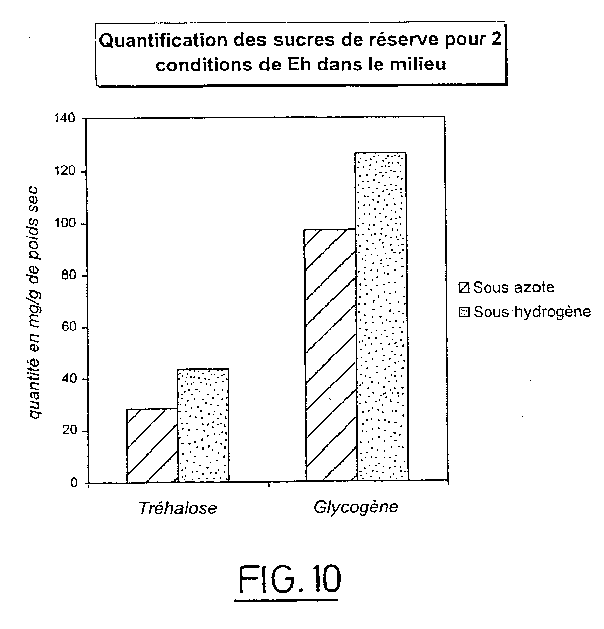

L'analyse quantitative de l'accumulation des sucres de réserve au sein de la cellule de levure rapportée sur la figure 10, montre qu'en présence de conditions réductrices selon l'invention, la souche synthétise des oligosaccharides de réserve en quantité plus importante qu'en conditions oxydantes en réponse à cette modification de l'environnement physico-chimique.

### EXEMPLE 5 : ETUDE SUR MILIEU JUS DE RAISIN.

On a utilisé une souche oenologique de *Saccharomyces cerevisiae* RC 212. La fermentation est menée en discontinu (batch) pendant 145 heures sur milieu jus de raisin contenant environ 165 g/l de sucres fermentescibles et la température est maintenue à 25°C.

Quatre conditions de Eh sont testées en utilisant :
- un réacteur sans bullage de gaz
- un réacteur avec un bullage azote
- un réacteur avec un bullage azote/hydrogène (conditions selon l'invention)
- un réacteur avec un bullage hydrogène (conditions selon l'invention).

On a prélevé les cellules pour le dosage du tréhalose et du glycogène au bout de 128 heures de fermentation alcoolique, quand la quasi-totalité des sucres fermentescibles a été consommée (environ 99.9%).

On a mesuré, dans le même temps, la viabilité des cellules au bleu de méthylène. Les échantillons prélevés sont ensuite conservés à 4°C sans précaution particulière si ce n'est qu'une partie des cellules est reprise dans de l'eau physiologique et l'autre partie dans le milieu vin dans lequel elles ont été prélevées. Un suivi au cours du temps de la viabilité a été réalisé.

### - Dosage du tréhalose et du glycogène :

Les résultats présentés correspondent à une moyenne de 2 répétitions.

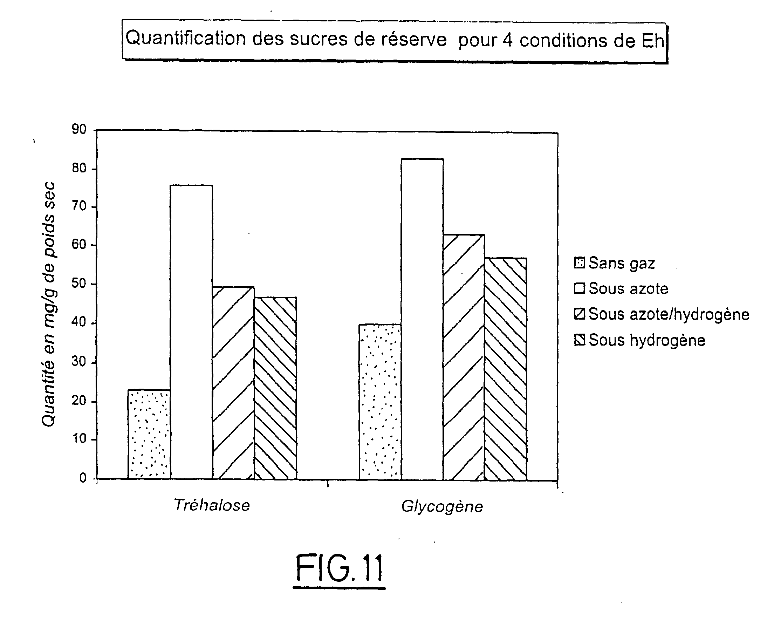

A partir de l'analyse quantitative illustrée sur la figure 11, on peut remarquer que la levure accumule plus de sucres de réserve lorsqu'elle est en conditions réductrices conformément à l'invention plutôt qu'en conditions oxydantes.

On remarque que cette accumulation est optimale sur le milieu placé sous azote, gaz non réducteur mais conduisant à un abaissement du potentiel redox du milieu par rapport à la valeur qu'il aurait eu en l'absence de gaz (azote).

### - Suivi de la viabilité au cours du temps:

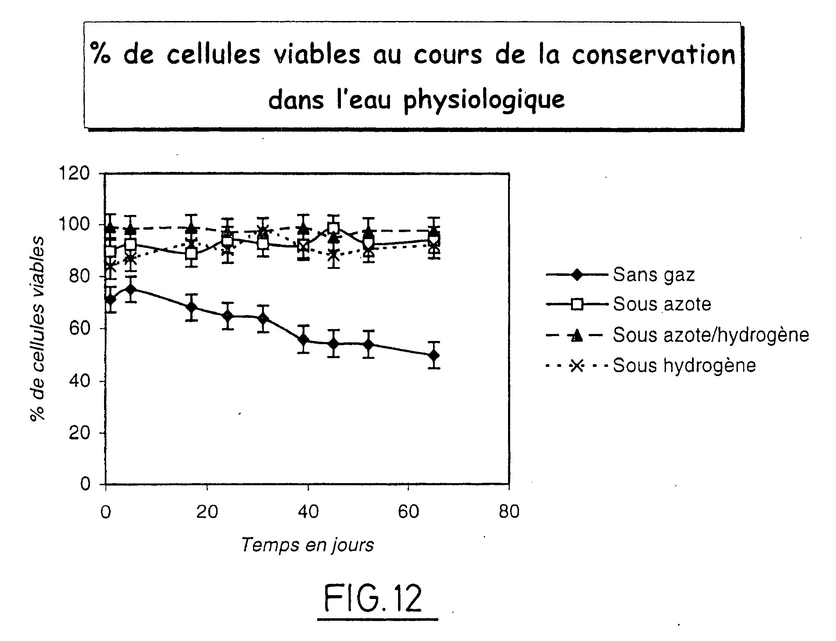

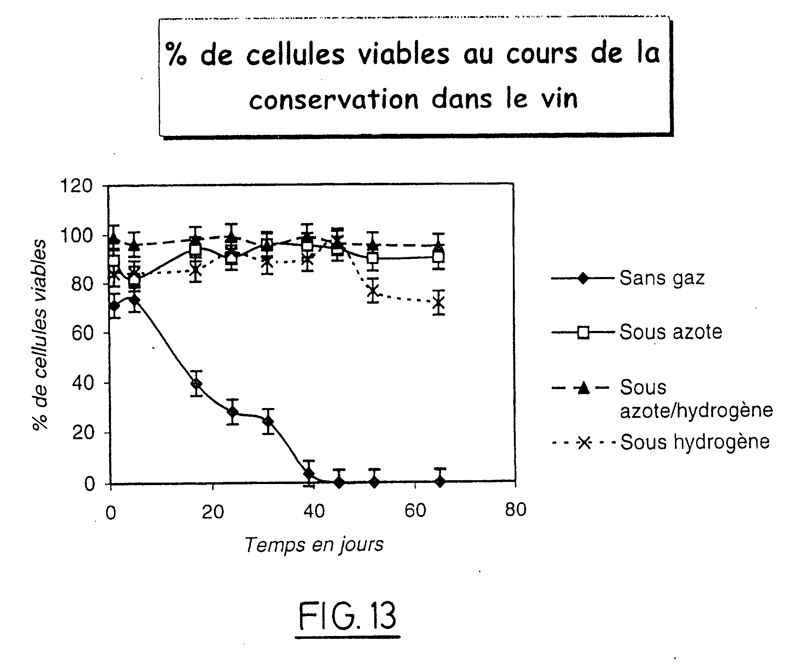

Aux vus des courbes données sur les figures 12 et 13, il est très intéressant de noter que la viabilité est optimale pour les cellules issues du réacteur placé sous azote/hydrogène. Dans ce cas, la valeur du Eh correspondant à ce mélange apparaît comme la plus optimale pour permettre une meilleure conservation des cellules de levure au cours du temps.

## Revendications

1. Procédé de culture de mioro-organismes :
- pour la production de ferments, notamment levains de panification, levures-aliments et extraits de levures, où les microorganismes sont situés dans le groupe constitué des espèces des genres *Saccharomyces, Candida* et *Kluyveromyces ;* et
- pour la production de boissons non gazeuses à degré alcoolique réduit, et, de boissons alcoolisées gazeuses, où les microorganismes sont situés dans le groupe constitué des espèces du genre *Saccharomyces,*
**caractérisé en ce que** l'on effectue ladite culture sous un gaz réducteur comprenant de l'hydrogène, afin d'abaisser le potentiel d'oxydoréduction du milieu de culture, par rapport à la valeur qu'il aurait sous air, toutes choses étant égales par ailleurs.

2. Procédé selon la revendication 1, **caractérisé en ce qu'**il est destiné à la production de boissons non gazeuses à degré alcoolique réduit par fermentation utilisant des levures en récipients ouverts, notamment en cuves, et **en ce que** l'on augmente la quantité de glycérol produite et l'on diminue la quantité d'éthanol produite par rapport à la valeur qu'elles prendraient sous air, toutes choses étant égales par ailleurs.

3. Procédé selon la revendication 2, **caractérisé en ce que** le rapport glycérol / éthanol est multiplié par un facteur 1,5 à 5. de préférence 2 à 4.

4. Procédé selon la revendication 1, **caractérisé en ce qu'**il est destiné à la production de ferments, notamment levains de panification, levures-aliments et extraits de levures, et **en ce qu'**il permet d' augmenter la quantité de sucres de réserve notamment tréhalose et glycogène, produite lors de ladite culture, par rapport à la valeur qu'elle aurait sous air, toutes choses étant égales par ailleurs.

5. Procédé selon l'une des revendications 1 à 4, **caractérisé en ce que** l'on effectue ladite culture des micro-organismes sous hydrogène.

6. Procédé selon l'une des revendications 1 à 4, **caractérisé en ce que** l'on effectue ladite culture sous un gaz formé d'un mélange d'hydrogène et d'azote.

7. Procédé selon l'une des revendications 1 à 4, **caractérisé en ce que** l'on effectue ladite culture sous un gaz contenant de l'hydrogène et de l'azote et un gaz complémentaire acceptable du point de vue de ladite culture.

8. Procédé selon la revendication 7, **caractérisé en ce que** le gaz complémentaire est choisi parmi les gaz inertes, notamment argon, hélium, et parmi l'oxygène, le dioxyde de carbone et le protoxyde d'azote et leurs mélanges en toutes proportions, de préférence parmi le dioxyde de carbone et l'oxygène ainsi que leurs mélanges.

9. Procédé selon l'une des revendications 1 à 4, **caractérisé en ce que** l'on effectue ladite culture sous un gaz contenant de l'hydrogène et du dioxyde de carbone.

## Claims

1. Process for culturing microorganisms:
- for the production of ferments, in particular breadmaking leavens, food yeasts and yeast extracts, where the microorganisms come from the group consisting of species of the genera *Saccharomyces, Candida* and *Kluyveromyces;* and
- for the production of CO₂-free beverages with a reduced level of alcohol and of carbonated alcoholic beverages, where the microorganisms come from the group consisting of species of the genus *Saccharomyces,*
**characterized in that** the said culturing is carried out under a reducing gas comprising hydrogen, in order to lower the oxidation/reduction potential of the culture medium, with respect to the value which it would have under air, all things otherwise being equal.

2. Process according to Claim 1, **characterized in that** it is intended for the production of CO₂-free beverages with a reduced level of alcohol by fermentation using yeasts in open vessels, in particular vats, and **in that** the amount of glycerol produced is increased and the amount of ethanol produced is reduced, with respect to the value which they would take under air, all things otherwise being equal.

3. Process according to Claim 2, **characterized in that** the glycerol/ethanol ratio is multiplied by a factor of 1.5 to 5, preferably of 2 to 4.

4. Process according to Claim 1, **characterized in that** it is intended for the production of ferments, in particular breadmaking leavens, food yeasts and yeast extracts, and **in that** it makes it possible to increase the amount of storage sugars, in particular trehalose and glycogen, produced during the said culturing, with respect to the value which it would have under air, all things otherwise being equal.

5. Process according to one of Claims 1 to 4, **characterized in that** the said culturing of the microorganisms is carried out under hydrogen.

6. Process according to one of Claims 1 to 4, **characterized in that** the said culturing is carried out under a gas formed of a mixture of hydrogen and nitrogen.

7. Process according to one of Claims 1 to 4, **characterized in that** the said culturing is carried out under a gas comprising hydrogen and nitrogen and an additional gas acceptable from the viewpoint of the said culturing.

8. Process according to Claim 7, **characterized in that** the additional gas is chosen from inert gases, in particular argon or helium, and from oxygen, carbon dioxide and nitrous oxide and their mixtures in all proportions, preferably from carbon dioxide and oxygen as well as their mixtures.

9. Process according to one of Claims 1 to 4, **characterized in that** the said culturing is carried out under a gas comprising hydrogen and carbon dioxide.

## Patentansprüche

1. Verfahren zum Kultivieren von Mikroorganismen:
- zur Erzeugung von Fermenten, insbesondere Hefen zur Brotbereitung, Nahrungsmittelhefen oder Hefeextrakten, in denen die Mikroorganismen in der Gruppe angeordnet sind, die von den Gattungen der Spezies *Saccharomyces, Candida* und *Kluyveromyces* gebildet ist; und
- zur Erzeugung von nicht kohlensäurehaltigen Getränken mit reduziertem Alkoholgehalt und von kohlensäurehaltigen alkoholischen Getränken, in denen die Mikroorganismen in der Gruppe angeordnet sind, die von den Gattungen der Spezies *Saccharomyces* gebildet ist,
**dadurch gekennzeichnet, dass** das Kultivieren unter einem reduzierenden Gas erfolgt, umfassend Wasserstoff, um das Oxyreduktionspotential des Kulturmediums im Vergleich mit dem Wert, den es unter Luft hätte, zu senken, wobei alle anderen Bedingungen gleich bleiben.

2. Verfahren nach Anspruch 1, **dadurch gekennzeichnet, dass** es für die Erzeugung von nicht kohlensäurehaltigen Getränken mit reduziertem Alkoholgehalt durch Fermentation bestimmt ist, die Hefen in offenen Behältern, insbesondere in Wannen, verwendet, und dass die erzeugte Glycerolmenge erhöht und die erzeugte Ethanolmenge verringert werden im Vergleich mit dem Wert, den sie unter Luft annehmen würden, wobei alle anderen Bedingungen gleich bleiben.

3. Verfahren nach Anspruch 2, **dadurch gekennzeichnet, dass** das Verhältnis Glycerol/Ethanol mit einem Faktor 1,5 bis 5, vorzugsweise 2 bis 4, multipliziert wird.

4. Verfahren nach Anspruch 1, **dadurch gekennzeichnet, dass** es für die Erzeugung von Fermenten, insbesondere Hefen zur Brotbereitung, Nahrungsmittelhefen und Hefeextrakten, bestimmt ist, und dass es eine Erhöhung der Reservezuckermenge, insbesondere Trehalose und Glykogen, die beim Kultivieren erzeugt wird, im Vergleich mit dem Wert, den sie unter Luft hätte, ermöglicht, wobei alle anderen Bedingungen gleich bleiben.

5. Verfahren nach einem der Ansprüche 1 bis 4, **dadurch gekennzeichnet, dass** das Kultivieren der Mikroorganismen unter Wasserstoff stattfindet.

6. Verfahren nach einem der Ansprüche 1 bis 4, **dadurch gekennzeichnet, dass** das Kultivieren unter einem Gas, das von einem Wasserstoff- und Stickstoffgemisch gebildet ist, erfolgt.

7. Verfahren nach einem der Ansprüche 1 bis 4, **dadurch gekennzeichnet, dass** das Kultivieren unter einem Gas, das Wasserstoff und Stickstoff und ein Ergänzungsgas, das aus Sicht des Kultivierens annehmbar ist, enthält, erfolgt.

8. Verfahren nach Anspruch 7, **dadurch gekennzeichnet, dass** das Ergänzungsgas unter den inerten Gasen ausgewählt wird, insbesondere Argon, Helium, und unter Sauerstoff, Kohlendioxid und Stickstoffprotoxid und ihren Gemischen in allen Verhältnissen, vorzugsweise unter Kohlendioxid und Sauerstoff sowie ihren Gemischen.

9. Verfahren nach einem der Ansprüche 1 bis 4, **dadurch gekennzeichnet, dass** das Kultivieren unter einem Gas erfolgt, das Wasserstoff und Kohlendioxid enthält.
